# EUROPEAN PATENT APPLICATION

(11) **EP 4 664 470 A1**
(43) Date of publication of application: **17.12.2025**
(21) Application number: 24752735.1
(22) Date of filing: 30.01.2024
(51) Int. Cl.: G16H 20/40

(54) **MODULAR TREATMENT PLANNING SYSTEM, SYSTEM CONSTRUCTION METHOD, COMPUTER SYSTEM, AND STORAGE MEDIUM**

(30) Priority: 09.02.2023 CN 202310100769
(71) Applicant: Neuboron Therapy System Ltd., Xiamen, Fujian 361026 (CN)
(72) Inventor: CHEN, Jiang, Nanjing, Jiangsu 211112 (CN)
(74) Representative: Gunzelmann, Rainer
(86) International application number: PCT/CN2024/074602
(87) International publication number: WO 2024/164879

(57) **Abstract**

The present disclosure relates to a modular treatment planning system, a method for constructing a modular treatment planning system, a computer device, and a storage medium. The system includes a core module configured to set up a system framework; an expansion module including a plurality of functional units that have different functions; and a module manager configured to call at least one functional unit, and load the called functional unit into the system framework, where the functional unit called by the module manager is integrated into the system framework to form an operation interface. According to the present disclosure, functional modules of the treatment planning system can be updated and expanded according to requirements. Through differentiated module configurations, a simple functional requirement of a naive user can be satisfied, complexity of an operation process can be reduced, and working efficiency can be improved. Moreover, an advanced functional requirement of an advanced user can be satisfied, and the complex functional modules can be implemented.

## Description

### TECHNICAL FIELD

The present disclosure relates to the technical field of therapeutic systems, and particularly relates to a modular treatment planning system, a method for constructing a modular treatment planning system, a computer system, and a storage medium.

### BACKGROUND

As atomic science develops, radiotherapy by using Cobalt-60, a linear accelerator, or an electronic beam, etc. has become a principal means for treating cancer. However, conventional photon therapy or electron therapy is limited by physical conditions of radioactive rays, so a large quantity of normal tissue in a path of a beam will be damaged when a tumor cell is killed. In addition, due to different degrees of sensitivity of tumor cells to radioactive rays, conventional radiotherapy generally has a poor effect on relatively radiation-resistant malignant tumors (such as glioblastoma multiforme and melanoma).

To reduce a radiation damage on normal tissue around a tumor, target therapy of chemotherapy is applied to the radiotherapy. Moreover, radiation sources having high relative biological effectiveness (RBE), for example, proton therapy, heavy particle therapy, and neutron capture therapy, are actively developed currently for high-radiation-resistant tumor cells. The neutron capture therapy combines the foregoing two concepts. For example, boron neutron capture therapy (BNCT) provides a better choice for cancer therapy than conventional radioactive rays through specific aggregation of boron-containing medicine in a tumor cell and in combination with precise beam control. As a novel and effective means for cancer therapy, the boron neutron capture therapy is drawing increasing attention, and gradually enters a clinical application phase from a research phase. No matter which therapy method is adopted, a treatment planning system is a core subsystem of the entire radiotherapy. Through the treatment planning system, transport of particles in a patient can be simulated by using a computer program, to compute dose distribution in the patient. Through statistical analysis, an appropriate and effective plan can be made. Thus, a radiation dose can be concentrated in a treatment target region of a patient as much as possible, and a dose in the normal tissue can be reduced.

However, in clinical practice, an entire therapeutic process includes a plurality of phases, such as acquisition of image information, determination of a treatment target region, delineation of normal tissue, generation of an initial radiation plan, simulated positioning, determination of a final radiation plan, positioning before radiation, implementation of radiation, and evaluation of a dose. Functions of treatment planning systems used for different radiation parts are different. In addition, different users have different requirements on system functions. Users expect to have an extremely simple function interface for clinical application, to avoid misoperation in use. Researchers expect to have a more advanced function to facilitate statistical analysis of data. In an actual application process, a data management system, a device control system, and so on are generally designed to be connected to or communicate with an external third-party system. To satisfy these different user requirements, a treatment planning system generally has an increasingly advanced function and an increasingly complex user interface.

### SUMMARY

To satisfy different user requirements and reduce a use threshold for a user, an objective of the present disclosure is to provide a modular treatment planning system, a method for constructing a modular treatment planning system, a computer system, and a storage medium, which can facilitate user customization, to overcome a design defect of an existing treatment planning system.

In a first aspect, the present disclosure provides a modular treatment planning system. The system includes:
a core module configured to set up a system framework;
an expansion module including a plurality of functional units that have different functions; and
a module manager configured to call at least one functional unit, and load the called functional unit into the system framework, where
the functional unit called by the module manager is integrated into the system framework to form an operation interface.

In an embodiment, the functional unit is one or more or all of a plan maker unit, a dose calculation unit, a display unit, an image data processing unit, a treatment report generation unit, a radiation dose measurement unit, and a radiation parameter correction unit.

In an embodiment, the plan includes one or more or all of radiation time, a radiation dose, a radiation angle, a radiation position, a neutron dose, and a photon dose that are obtained by using the expansion module.

In an embodiment, the module manager calls the expansion module by using a dynamic link library.

In an embodiment, the expansion module, the system framework, the core module, or the module manager is constructed by using a dynamic link library, or includes a component constructed by using the dynamic link library.

In an embodiment, the module manager includes an interface unit, and the module manager is connected to the expansion module by using the interface unit.

In an embodiment, the plan generation unit performs steps including:
performing image data processing, which includes at least steps of determining a region of interest (ROI) and performing tissue type definition on the ROI;
acquiring a beam parameter, where the beam parameter includes at least a beam diameter and beam intensity; and
setting a Monte Carlo control parameter for simulation.

In an embodiment, the display unit displays one or more or all of the radiation time, the radiation dose, the radiation angle, the radiation position, the neutron dose, and the photon dose that are generated by the expansion module.

In an embodiment, the treatment report generation unit generates a treatment report, and the treatment report includes all or some computation results generated by the expansion module.

In a second aspect, the present disclosure further provides a method for constructing a modular treatment planning system. The method includes:
configuring a core module, a system framework, an expansion module, and a module manager in a treatment planning system;
setting up the system framework by the core module;
calling the expansion module, and loading the expansion module into the system framework by the module manager; and
integrating the expansion module called by the module manager into the system framework to form an operation interface.

In an embodiment, the expansion module includes a plurality of functional units that have different functions, and the module manager calls at least one functional unit.

In an embodiment, the expansion module includes at least one or more or all of a plan generation unit, a dose calculation unit, a display unit, an image data processing unit, a treatment report generation unit, a radiation dose measurement unit, and a radiation parameter correction unit.

In a third aspect, the present disclosure further provides a computer device. The computer device includes a memory and a processor. The memory stores a computer program. The processor, when executing the computer program, implements steps as follows:
configuring a core module, a system framework, an expansion module, and a module manager in a treatment planning system;
setting up the system framework by the core module;
calling the expansion module, and loading the expansion module into the system framework by the module manager; and
integrating the expansion module called by the module manager into the system framework to form an operation interface.

In a fourth aspect, the present disclosure further provides a computer-readable storage medium. The computer-readable storage medium stores a computer program. The computer program, when executed by a processor, implements steps as follows:
configuring a core module, a system framework, an expansion module, and a module manager in a treatment planning system;
setting up the system framework by the core module;
calling the expansion module, and loading the expansion module into the system framework by the module manager; and
integrating the expansion module called by the module manager into the system framework to form an operation interface.

According to the modular treatment planning system and the method for constructing a modular treatment planning system mentioned above, a software architecture of a treatment planning system based on a dynamic link library is designed. The entire treatment planning system is constructed in a layered manner. Thus, functional modules of the treatment planning system can be conveniently updated and expanded. Through differentiated module configurations, a simple functional requirement of a naive user can be satisfied, complexity of an operation process can be reduced, and working efficiency can be improved. Moreover, an advanced functional requirement of an advanced user can be satisfied, and the complex functional modules can be implemented. Moreover, personalized customization of functions can be performed according to an actual application scenario. Through one-time configuration, the treatment planning system is automatically loaded based on a corresponding functional module during startup, such that operation is simple, and an automation degree is high. Further, a practical solution is provided for different users in different application scenarios.

### BRIEF DESCRIPTION OF THE DRAWINGS

FIG. 1 is a block diagram of a treatment planning system according to an embodiment;
FIG. 2 is a schematic diagram of a boron neutron capture reaction;
FIG. 3 shows a formula of a ¹⁰B(n,α)⁷Li neutron capture nuclear reaction;
FIG. 4 is a block diagram of a neutron capture therapy system according to an embodiment;
FIG. 5 is a schematic diagram of functional units of a treatment planning system according to an embodiment; and
FIG. 6 is a flowchart of a method for constructing a treatment planning system according to an embodiment.

Description of reference numerals:
100 boron neutron capture therapy system, 10 neutron beam radiation apparatus, 20 treatment planning system, 30 control system, 200 irradiated body, 21 core module, 22 system framework, 23 module manager, 24 expansion module, 241 plan generation unit, 242 dose calculation unit, and 243 treatment report generation unit.

### DETAILED DESCRIPTION

To make the objectives, technical solutions, and advantages of the present disclosure clearer, the present disclosure is described in further detail in combination with the accompanying drawings and the embodiments. It should be understood that the particular embodiments described herein are merely used for explaining the present disclosure rather than limiting the present disclosure.

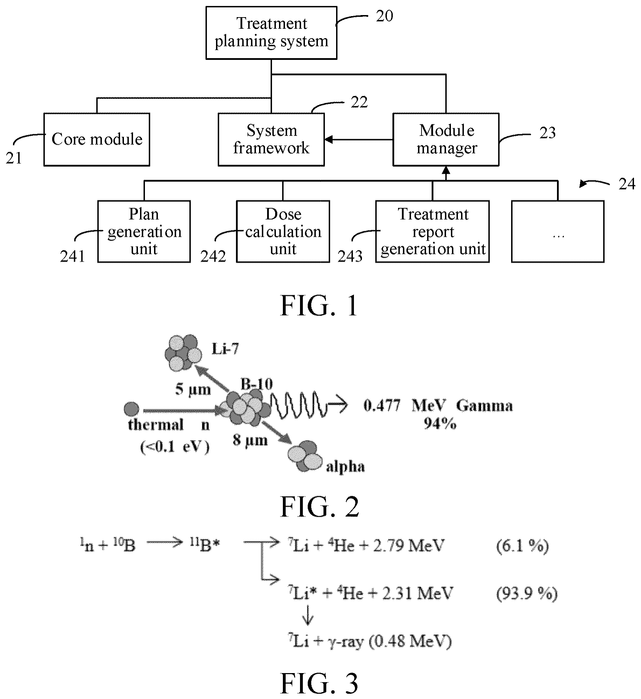

As shown in FIG. 1, an embodiment provides a modular treatment planning system 20. The entire treatment planning system 20 is set up in two layers. The first layer includes a core module 21, a system framework 22, and a module manager 23. The modular treatment planning system in the embodiment can be applied to the field of radiotherapy. In the embodiment, neutron capture therapy, particularly, boron neutron capture therapy, is taken as an example. Basic knowledge of the boron neutron capture therapy is briefly introduced below.

As an effective means for cancer therapy, the neutron capture therapy is gradually more applied in recent years, and the boron neutron capture therapy is the most common therapy. Neutrons for the boron neutron capture therapy may be provided by a nuclear reactor or an accelerator. In the embodiment, with accelerator boron neutron capture therapy as an example, basic components of the accelerator boron neutron capture therapy generally include an accelerator for accelerating charged particles (such as protons or deuterons), a target material, a heat removal system, and a beam shaper. Neutrons are generated by accelerating the charged particles and the metal target material. An appropriate nuclear reaction is selected according to a neutron yield and neutron energy which are required, energy and an electric current of the accelerated charged particles which can be provided, and physical and chemical properties of the metal target material. Specifically, ⁷Li(p,n)⁷Be and ⁹Be(p,n)⁹B are nuclear reactions commonly discussed. Both of the two reactions are endothermic reactions. Energy thresholds of the two nuclear reactions are 1.881 MeV and 2.055 MeV respectively. An ideal neutron source for the boron neutron capture therapy is an epithermal neutron in an energy level of keV. Thus, theoretically, if a lithium metal target material is bombarded by using a proton having energy merely slightly greater than a threshold, a neutron having relatively low energy can be generated and used in the clinic without too much slowing processing. However, a section of a Li metal target material and a section of a Be metal target material on which a proton having threshold energy acts are not high. To generate a sufficiently large flux of neutrons, a proton having relatively high energy is generally used to initiate a nuclear reaction.

The boron neutron capture therapy (BNCT) is to generate two heavily charged particles ⁴He and ⁷Li through a ¹⁰B(n,α)⁷Li neutron capture and nuclear fission reaction by using a high capture section of a boron-10 (10B)-containing medicine for a thermal neutron. With reference to FIG. 2 and FIG. 3, a schematic diagram of a boron neutron capture reaction and a 10B(n,α)7Li neutron capture nuclear reaction equation are shown in the figures respectively. The two charged particles have average energy of approximately 2.33 MeV, high linear energy transfer (LET), and a short firing range. Linear energy transfer and a firing range of an α particle are 150 keV/µm and 8 µm respectively, and linear energy transfer and a firing range of a ⁷Li heavy charged particle are 175 keV/µm and 5 µm respectively. A total firing range of the two particles is approximately equal to the size of a cell. Thus, radiation damage to a living body can be limited at a cell level. When the boron-containing medicine selectively accumulates in a tumor cell, by matching an appropriate neutron radiation source, the tumor cell can be killed without causing too much damage to normal tissue.

A collision trajectory and energy distribution of a nuclear particle in a three-dimensional space inside an irradiated target can be accurately simulated through the Monte Carlo method. In the neutron capture therapy, to simulate an absorbed dose of a human body under a radiation condition to help doctor generate a plan, various types of processing are generally required to be performed on a medical image through a computer technology to establish an accurate lattice model required by Monte Carlo software, then simulation and computation are performed in combination with the Monte Carlo software. Medical image data may be magnetic resonance imaging (MRI) data, computed tomography (CT) data, positron emission tomography (PET) data, PET-CT data, or X-Ray imaging data. In the embodiment, description is performed based on the CT data, and the file format of the CT data is generally digital imaging and communications in medicine (DICOM). However, a person skilled in the art well knows that other medical image data can be used. If the medical image data can be converted into a three-dimensional voxel model, the medical image data can be applied to the modular treatment planning system and the method for constructing a modular treatment planning system in the present disclosure.

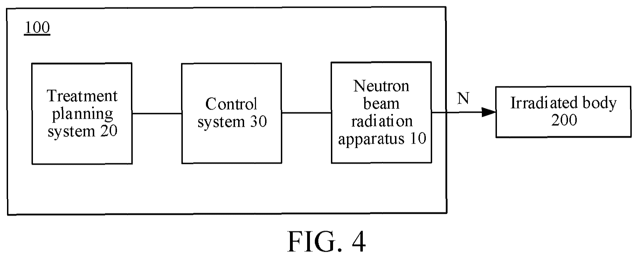

With reference to FIG. 4, a schematic structural diagram of a boron neutron capture therapy system is shown in FIG. 4. The boron neutron capture therapy system 100 includes a neutron beam radiation apparatus 10, a treatment planning system 20, and a control system 30. The neutron beam radiation apparatus 10 is configured to generate a treatment neutron beam N, and irradiate the treatment neutron beam N to an irradiated body 200 to form an irradiated part. The treatment planning module 20 performs dose simulation and computation according to medical image data of the irradiated part and generates a plan. In the plan, the position of the irradiated part relative to the neutron beam radiation apparatus 10 and corresponding radiation time during radiotherapy are determined. After the irradiated body 200 is positioned according to the position determined according to the plan, treatment can be started. The control module 30 calls the plan corresponding to the current irradiated body 200 from the treatment planning module 20, and controls radiation of the neutron beam radiation apparatus 10 according to the plan. The control module 30 may further receive other data information, such as data of the neutron beam radiation apparatus 10 and data of the irradiated body 200.

A core module 21 provides a basic function for the treatment planning system 20 and is necessary in all application scenarios. The core module 21 is configured to set up a system framework 22. Specifically, when the treatment planning system 20 is set up based on a computer operating system, the core module 21 includes basic functions of the computer operating system, such as a power-on function, a standby function, a software loading function, a data storage function, and a basic network communication function. The system framework 22 is an interface composition of the treatment planning system 20. The system framework 22 may be set up in the core module 21 in the form of software.

The treatment planning system 20 may further include an expansion module 24. The expansion module 24 includes a plurality of functional units that have different functions. The module manager 23 is configured to call at least one functional unit, and load the called functional unit into the system framework 22. In the embodiment, the expansion module 24 refers to a set of several functional units. The functional units are written in a computer language. The expansion module 24 may be entirely or partially preset in the treatment planning system 20 at the beginning, or may be obtained from the outside through some communication methods. The functional units included in the expansion module 24 may be loaded on a display interface according to a requirement of a user. Further, the module manager 23 includes an interface unit. The module manager 23 is connected to the expansion module 24 by using the interface unit.

Further, an entire framework is automatically constructed according to module composition. The module manager 23 configures a related module according to a requirement of an application user. The related module is automatically loaded in the system framework 22. A requirement of a user may be obtained at the beginning. The treatment planning system 20 automatically configures a corresponding expansion module 24 in the background by using the obtained requirement of the user and automatically loads the expansion module in the system framework 22.

The entire treatment planning system 20 is constructed through an object-oriented method. During construction, information interaction between data of the treatment planning system and a peripheral device in an entire BNCT treatment flow is considered, and a related interface is reserved. The treatment planning system 20 is constructed based on a modular manner. Modules are all implemented by a dynamic link library (or dynamic-link library, DLL). The dynamic link library is a method of the Microsoft Corporation for sharing a function library in a Microsoft Windows operating system. Such dynamic linking provides a method, through which a process can call a function that does not belong to executable code of the process. Executable code of a function is in a DLL file. The DLL includes one or more functions that have been compiled, linked, and stored separately from processes that use the functions. The DLL further helps to share data and resources. A plurality of applications may simultaneously access content of a single DLL copy in a memory. An update can be more easily applied to each module by using the dynamic link library, without affecting other portions of the program. The module manager 23 calls the expansion module 24 by using the dynamic link library. The expansion module 24, the system framework 22, the core module 21, or the module manager 23 is set up by using the dynamic link library, or includes a component set up by using the dynamic link library. Linking refers to adding code (which is generally a file having a suffix name of .lib or .a) of an external function into an executable file. The method of adding an external function library into an executable file through copying is referred to as static linking. In addition to the static linking, linking further includes dynamic linking. The dynamic linking refers to dividing modules of a program to form independent files rather than statically linking the modules together. In brief, object files constituting a program are not linked, and are linked when a program is required to be run. The dynamic link library has the following advantages. Fewer resources are used, specifically, when a plurality of programs use the same function library, the DLL can reduce theduplication amount of code loaded in a magnetic disk and a physical memory. A modular system structure is promoted, specifically, the DLL helps to promote development of a modular program, and develop a large program that is required to be provided in a plurality of language versions or a program that is required to have a modular system structure. Deployment and installation are simplified, specifically, when a function in the DLL is required to be updated or repaired, a link between a program and the DLL is not required to be reestablished when the DLL is deployed and installed. In addition, if a plurality of programs use the same DLL, the plurality of programs all benefits from the update or repair.

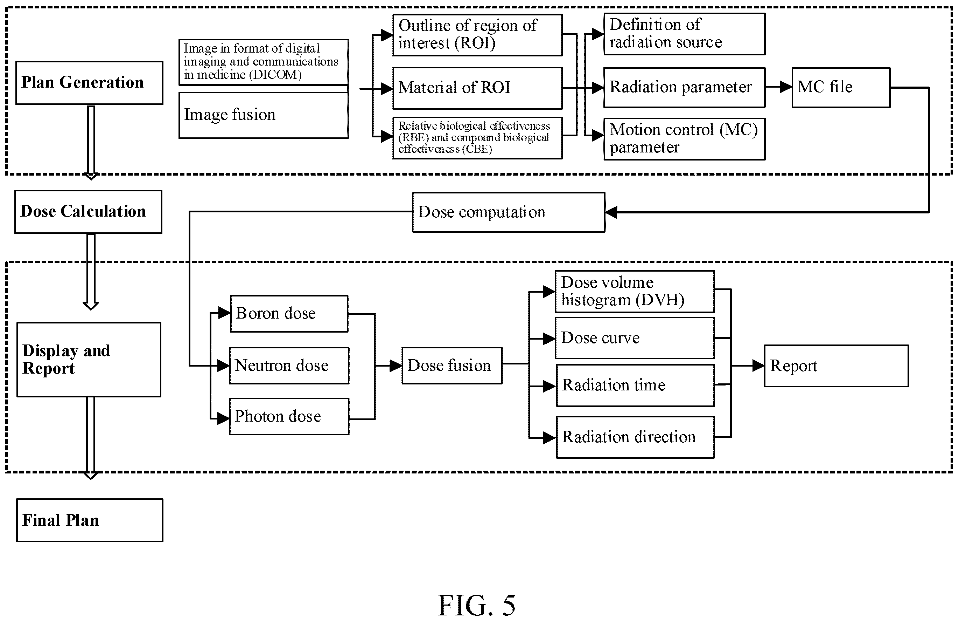

As shown in FIG. 5, a functional unit of the expansion module 24 is one of a plan generation unit 241, a dose calculation unit 242, a display unit, an image data processing unit, a treatment report generation unit 243, a radiation dose measurement unit, and a radiation parameter correction unit (not shown in the figure).

The plan generation unit 241 performs steps including: image data processing is performed, which includes at least steps that a region of interest (ROI) is determined and tissue type definition is performed on the ROI; a beam parameter is acquired, where the beam parameter includes at least a beam diameter and beam intensity; and a Monte Carlo control parameter is set for simulation. The image data processing by the plan generation unit 241 may include: an ROI is automatically determined according to image data of a patient, and tissue type definition is automatically performed on the ROI. The image data processing by the plan generation unit 241 may alternatively include: an ROI determined by a user is acquired, and a tissue type definition is performed on the ROI. Further, the step that tissue type definition is performed on the ROI is to define material element composition, a corresponding relative biological effectiveness (RBE) value, and a corresponding compound biological effectiveness (CBE) value on each ROI. In the step that a beam parameter is acquired, the plan generation unit 241 acquires a parameter inputted by a user, or automatically selects a corresponding parameter. The above beam parameter further includes a beam direction, a beam exit position, beam energy, etc.

An algorithm and a program that are related to the Monte Carlo method are set in the dose calculation unit 242, and are configured to compute a dose according to a Monte Carlo control parameter generated by the plan generation unit 241, or acquire a parameter inputted by a user into the dose calculation unit 242, and obtain a dose result.

The display unit is configured to display data involved in the treatment planning system 20, and may include input parameters, intermediate computation results, and output results of all functional units. The user may select a type of data displayed by the display unit. Corresponding data is loaded into the display unit according to a requirement. For example, in addition to a physical dose and a biologically equivalent dose, individual dose components (a boron dose, a neutron dose, and a photon dose) can also be displayed. The user can adjust and determine the radiation time according to the biologically equivalent dose and a dose limiting condition. The display unit displays one or more or all of radiation time, a radiation dose, a radiation angle, a radiation position, a neutron dose, a photon dose, a dose curve, and a dose volume histogram (DVH) that are generated by the expansion module. The radiation dose measurement unit may detect the radiation dose in real time by being connected to an external sensor, provide a corresponding signal indication, and transmit the corresponding signal indication to the display unit to display related dose data. The radiation dose measurement unit may be integrated into the display unit.

The image data processing unit is configured to perform processing on image data inputted into the treatment planning system 20. The image data includes a one-dimensional, two-dimensional, or three-dimensional image and a related parameter. The image is mainly acquired by an external scanning device and transmitted to the treatment planning system 20. Through the processing on the image data by the image data processing unit, the plan generation unit 241 can automatically recognize and generate a result such that a parameter of a body of a patient can be intuitively obtained. Further, the processing includes but is not limited to image format recognition, display effect recognition, and image recognition, etc.

The treatment report generation unit 243 is configured to generate a treatment report based on treatment information required by a user, and provide a parameter required for subsequent treatment. The treatment report generation unit 243 may include some or all data displayed by the display unit. The treatment report generation unit 243 may provide a parameter required for treatment automatically or through selection by the user. The treatment report generation unit 243 may be integrated with the display unit to form a display and report unit.

The radiation parameter correction unit may obtain the radiation parameter in real time by being connected to an external sensor and provide a corresponding parameter to another functional unit.

The functional unit of the expansion module 24 may further include a transmission unit. The transmission unit may upload data such as the treatment report to a server-side of an external control system by using a network as a plan finally received by the patient. The server may be implemented by using an independent server or a server cluster composed of a plurality of servers.

All or some of the modules of the above treatment planning system may be implemented by software, hardware, or their combination. The above modules may be embedded in or independent of a processor of a computer device in a form of hardware or may be stored in a memory of a computer device in a form of software such that the processor can call and perform operations corresponding to the above modules.

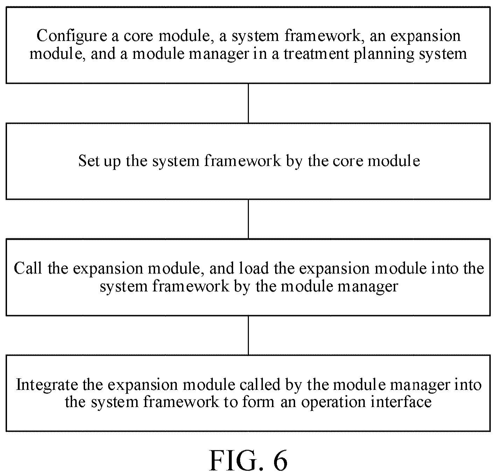

As shown in FIG. 6, another embodiment provides a method for constructing a modular treatment planning system. The method may be applied to application environments shown in FIG. 1 and FIG. 4, and specifically may be applied to construct a treatment planning system 20. The constructed treatment planning system 20 may be but is not limited to a personal computer, a notebook computer, a smartphone, a tablet computer, an internet of things device, and a portable wearable device. The internet of things device may be a smart speaker, a smart television, a smart air conditioner, an intelligent in-vehicle device, etc. The portable wearable device may be a smart watch, a smart band, a head-mounted device, etc. As shown in FIG. 6, the method for constructing a modular treatment planning system includes steps as follows:
a core module 21, a system framework 22, an expansion module 24, and a module manager 23 are configured in a treatment planning system 20;
the system framework 22 is set up by the core module 21;
the expansion module 24 is called, and the expansion module 24 is loaded into the system framework 22 by the module manager 23; and
the expansion module 24 called by the module manager 23 is integrated into the system framework 22 to form an operation interface.

According to the modular treatment planning system and the method for constructing a modular treatment planning system mentioned above, a software architecture of a treatment planning system based on a dynamic link library is designed, has a feature of layered construction, and includes a basic module layer and an expansion module layer. Since the dynamic link library is loaded during work, and a hot swap is supported, a dynamic link library can be independently updated without recompiling entire software. Accordingly, functional modules of the treatment planning system can be conveniently updated and expanded. Corresponding dynamic link libraries can be provided to be automatically adapted to external devices of different manufacturers. Through differentiated module configurations, a simple functional requirement of a naive user can be satisfied, complexity of an operation process can be reduced, and working efficiency can be improved. Moreover, an advanced functional requirement of an advanced user can be satisfied, and the complex functional modules can be implemented. Moreover, personalized customization of functions can be performed according to an actual application scenario. Through one-time configuration, the treatment planning system is automatically loaded based on a corresponding functional module during startup, such that operation is simple, and an automation degree is high. Three main modules, that is, a plan generation module, a dose calculation module, and a display and report module, are set up in a typical boron neutron capture therapy flow. An entire boron neutron capture therapy flow can be conveniently and efficiently completed.

It should be understood that although the steps in the flowcharts involved in the above embodiments are shown successively according to indications of arrows, the steps are not certainly performed successively in the sequence indicated by the arrows. Unless explicitly stated herein, these steps are not strictly limited to the sequence in which they are executed, and can be executed in other sequences. Moreover, at least some steps in the flowcharts involved in the above embodiments can include a plurality of steps or a plurality of stages. These steps or stages are not certainly performed at the same time, but can be performed at different time. These steps or stages are not certainly performed in sequence, but can be performed in turn or alternatively with other steps or at least some steps or stages of other steps.

In an embodiment, a computer device is provided. The computer device may be a server. The computer device includes a processor, a memory, and a network interface which are connected by a system bus. The processor of the computer device is configured to provide a computation capability and a control capability. The memory of the computer device includes a non-volatile storage medium and an internal memory. The non-volatile storage medium stores an operating system, a computer program, and a database. The internal memory provides an environment for running of the operating system and the computer program in the non-volatile storage medium. The database of the computer device is configured to store data generated by the above functional modules. The network interface of the computer device is configured to be in communication with an external terminal through a network connection. The computer program, when executed by the processor, implements a method for constructing a modular treatment planning system.

In an embodiment, a computer device is provided. The computer device may be a terminal. The computer device includes a processor, a memory, a communication interface, a display screen, and an input apparatus that are connected by using a system bus. The processor of the computer device is configured to provide a computation capability and a control capability. The memory of the computer device includes a non-volatile storage medium and an internal memory. The non-volatile storage medium stores an operating system and a computer program. The internal memory provides an environment for running of the operating system and the computer program in the non-volatile storage medium. The communication interface of the computer device is configured to be in communication with an external terminal through a wired method or a wireless method. The wireless method may be implemented by using WIFI, a mobile cellular network, near field communication (NFC), or other technology. The computer program, when executed by the processor, implements a method for constructing a modular treatment planning system. The display screen of the computer device may be a liquid crystal display screen or an e-ink display screen. The input apparatus of the computer device may be a touch layer covering the display screen, or may be a key, a trackball, or a touchpad arranged on a housing of the computer device, or may be an external keyboard, touchpad, or mouse, etc.

A person skilled in the art can understand that the above structures are merely some structures related to the solution of the present disclosure, and does not constitute a limitation on the computer device to which the solution of the present disclosure is applied. A specific computer device may include more or less components than those shown in the figure, or combine some components, or have different component arrangements.

In an embodiment, a computer device is provided. The computer device includes a memory and a processor. The memory stores a computer program. The processor, when executing the computer program, implements the above method for constructing a modular treatment system.

In an embodiment, a computer-readable storage medium is provided. The computer-readable storage medium stores a computer program. The computer program, when executed by a processor, implements the above method for constructing a modular treatment system.

It should be noted that user information (including but not limited to user equipment information, user personal information, etc.) and data (including but not limited to data for analysis, stored data, and displayed data) involved in the present disclosure are information and data authorized by a user or fully authorized by all parties.

A person of ordinary skill in the art can understand that all or some of flows of the method in the above embodiments may be implemented by a computer program instructing related hardware. The computer program may be stored in a non-volatile computer-readable storage medium. The computer program, when executed, may include the flows of the above embodiments of the method. Any reference to a memory, a database, or other media used in the embodiments provided in the present disclosure may include at least one of a non-volatile memory and a volatile memory. The non-volatile memory may include a read-only memory (ROM), a magnetic tape, a floppy disk, a flash memory, an optical memory, a high-density embedded non-volatile memory, a resistive random access memory (ReRAM), a magneto resistive random access memory (MRAM), a ferroelectric random access memory (FRAM), a phase change memory (PCM), a graphene memory, etc. The volatile memory may include a random access memory (RAM) or an external cache memory. By way of illustration and not limitation, the RAM may be in a variety of forms, such as a static random access memory (SRAM) or a dynamic random access memory (DRAM). The database involved in the embodiments provided in the present disclosure may include at least one of a relational database and a non-relational database. The non-relational database may include a distributed database based on a block chain, but is not limited thereto. The processor involved in the embodiments provided in the present disclosure may be a general-purpose processor, a central processing unit, a graphic processing unit, a digital signal processor, a programmable logic unit, a data processing logic unit based on quantum computation, etc., but is not limited thereto.

Technical features of the above embodiments can be combined with one another. To make description concise, not all possible combinations of the technical features in the above embodiments are described. However, the combinations of these technical features should be considered as falling within the scope of recitation of the description provided that no conflict exists.

The above embodiments only express some implementations of the present disclosure, which are specifically described in detail but cannot be construed as a limitation on the patent scope of the present disclosure. It should be noted that several variations and improvements can be made by a person of ordinary skill in the art without departing from the concept of the present disclosure, and should all fall within the scope of protection of the present disclosure. Thus, the scope of protection of the present disclosure should be subject to the appended claims.

## Claims

1. A modular treatment planning system, comprising:
a core module configured to set up a system framework;
an expansion module comprising a plurality of functional units that have different functions; and
a module manager configured to call at least one functional unit, and load the called functional unit into the system framework, wherein
the functional unit called by the module manager is integrated into the system framework to form an operation interface.

2. The modular treatment planning system according to claim 1, wherein the functional unit is one or more or all of a plan generation unit, a dose calculation unit, a display unit, an image data processing unit, a treatment report generation unit, a radiation dose measurement unit, and a radiation parameter correction unit.

3. The modular treatment planning system according to claim 1, wherein the plan comprises one or more or all of radiation time, a radiation dose, a radiation angle, a radiation position, a neutron dose, and a photon dose that are obtained by using the expansion module.

4. The modular treatment planning system according to claim 1, wherein the module manager calls the expansion module by using a dynamic link library.

5. The modular treatment planning system according to claim 1, wherein the expansion module, the system framework, the core module, or the module manager is constructed by using a dynamic link library, or comprises a component constructed by using the dynamic link library.

6. The modular treatment planning system according to claim 1, wherein the module manager comprises an interface unit, and the module manager is connected to the expansion module by using the interface unit.

7. The modular treatment planning system according to claim 2, wherein the plan generation unit performs steps comprising:
performing image data processing, which comprises at least steps of determining a region of interest (ROI) and performing tissue type definition on the ROI;
acquiring a beam parameter, wherein the beam parameter comprises at least a beam diameter and beam intensity; and
setting a Monte Carlo control parameter for simulation.

8. The modular treatment planning system according to claim 3, wherein the display unit displays one or more or all of the radiation time, the radiation dose, the radiation angle, the radiation position, the neutron dose, and the photon dose that are generated by the expansion module.

9. The modular treatment planning system according to claim 2, wherein the treatment report generation unit generates a treatment report, and the treatment report comprises all or some computation results generated by the expansion module.

10. A method for constructing a modular treatment planning system, comprising:
configuring a core module, a system framework, an expansion module, and a module manager in a treatment planning system;
setting up the system framework by the core module;
calling the expansion module, and loading the expansion module into the system framework by the module manager; and
integrating the expansion module called by the module manager into the system framework to form an operation interface.

11. The method for constructing a modular treatment planning system according to claim 10, wherein the expansion module comprises a plurality of functional units that have different functions, and the module manager calls at least one functional unit.

12. The method for constructing a modular treatment planning system according to claim 10, wherein the expansion module comprises at least one or more or all of a plan generation unit, a dose calculation unit, a display unit, an image data processing unit, a treatment report generation unit, a radiation dose measurement unit, and a radiation parameter correction unit.

13. The method for constructing a modular treatment planning system according to claim 10, wherein the treatment planning system obtains, by using the expansion module, one or more or all of radiation time, a radiation dose, a radiation angle, a radiation position, a neutron dose, and a photon dose.

14. The method for constructing a modular treatment planning system according to claim 10, wherein the module manager calls the expansion module by using a dynamic link library.

15. The method for constructing a modular treatment planning system according to claim 10, wherein the module manager comprises an interface unit, and the module manager is connected to the expansion module by using the interface unit.

16. A computer device, comprising a memory and a processor, wherein the memory stores a computer program, and the processor, when executing the computer program, implements steps of the method according to any one of claims 10 to 15.

17. A computer-readable storage medium, storing a computer program, wherein the computer program, when executed by a processor, implements the method according to any one of claims 10 to 15.
